Europäisches Patentamt

European Patent Office

Office européen des brevets

(11) Veröffentlichungsnummer: **0 328 760**

**A2**

(12) **EUROPÄISCHE PATENTANMELDUNG**

(21) Anmeldenummer: 88120478.8

(51) Int. Cl.⁴: **A61M 25/00**

(22) Anmeldetag: 07.12.88

(30) Priorität: 18.02.88 DE 3804944

(43) Veröffentlichungstag der Anmeldung:
23.08.89 Patentblatt 89/34

(84) Benannte Vertragsstaaten:
AT BE CH DE ES FR GB GR IT LI LU NL SE

(71) Anmelder: **B. Braun Melsungen AG**
**Carl-Braun Strasse**
**D-3508 Melsungen(DE)**

(72) Erfinder: **Haindl, Hans-Günter**
**Schoene Aussicht 4**
**D-3508 Melsungen(DE)**
Erfinder: **Fuchs, Jürgen**
**Wolfhager Strasse 45**
**D-3501 Emstal(DE)**

(74) Vertreter: **Selting, Günther, Dipl.-Ing. et al**
**Patentanwälte von Kreisler, Selting, Werner**
**Deichmannhaus am Hauptbahnhof**
**D-5000 Köln 1(DE)**

(54) **Vorrichtung zum Einfädeln eines Führungsdrahtes in einen Katheter.**

(57) Die Erfindung zeichnet sich aus durch ein längsteilbares Kopfstück (11) mit einem axial durchgehenden geraden Kanal (14), der einen zylindrischen Abschnitt (31) aufweist, in welchen die Spitze (41) des Katheters (40) lösbar eingeklemmt ist und an den sich ein trichterförmiger Abschnitt (30) mit nach außen gerichteter größerer Öffnung anschließt.

Ein solches Kopfstück (11) erleichtert das Einfädeln dadurch, daß das extrakorporale Ende eines durch eine Punktionskanüle in eine Körperhöhle eingeführten Führungsdrahtes (10) bei der Annäherung des Kopfstückes (11) gegen die Schrägfläche des trichterförmigen Abschnittes (30) des Kanals (14) trifft und zwangläufig in die koaxial in dem Kanal (14) gehaltene Spitze (41) des Katheters (40) gelenkt wird.

FIG.2

EP 0 328 760 A2

## Vorrichtung zum Einfädeln eines Führungsdrahtes in einen Katheter

Die Erfindung bezieht sich auf eine Vorrichtung zum Einfädeln eines Führungsdrahtes in einen Katheter.

Um bei der Einbringung von flexiblen Kathetern in Blutgefäße oder andere Körperhöhlen die Traumatisierung des Gewebes gering zu halten, wird z.B. nach der Seldinger-Technik mit einer dünnen Stahlkanüle punktiert, durch die Stahlkanüle ein Führungsdraht in das Zielgebiet vorgeschoben und dann nach Entfernung der Stahlkanüle über den Führungsdraht der Katheter in die Korperhöhle eingeschoben. Ein solches Vorgehen ist bei der Katheterverlegung an sich günstig; wirft jedoch Probleme dadurch auf, daß der hantierende Arzt beim Zusammenfügen von Katheterspitze und Führungsdrahtende sehr genau zielen muß, was gutes räumliches Sehvermögen und eine ruhige Hand des Anwenders verlangt. Vor allem bei dünnen und durchsichtigen Kathetern wird das Auffädeln häufig außerordentlich erschwert, weil die Durchmesser von Katheter und Führungsdraht einander weitgehend angepaßt sind und Einfädelspielraum fehlt.

Der Erfindung liegt die Aufgabe zugrunde, eine Vorrichtung zu schaffen, die das Auffädeln eines Katheters auf einen Führungsdraht erleichtert.

Diese Aufgabe wird erfindungsgemäß gelöst durch ein längsteilbares Kopfstück mit einem axial durchgehenden geraden Kanal, der an einem Ende einen zylindrischen Abschnitt aufweist, in welchem die Spitze des Katheters lösbar eingeklemmt ist und der am anderen Ende mit einem trichterförmigen Abschnitt versehen ist, dessen größere Öffnung nach außen gerichtet ist.

Ein solches Kopfstück, dessen eines Ende mit der Spitze des Katheters, insbesondere eines langen flexiblen Katheters, klemmend verbunden ist, dient als Einführhilfe beim Einfädeln eines Führungsdrahtes in den Katheter. Das Einfädeln wird dadurch erleichtert, daß das extrakorporale Ende eines durch eine Punktionskanüle in eine Körperhöhle eingeführten Führungsdrahtes, z.B. einer Seldinger-Spirale oder einer anderen langgestreckten Führungssonde, bei der Annäherung des Kopfstückes gegen die Schrägfläche des trichterförmigen Abschnittes des Kanales trifft und zwangläufig in die koaxial in dem Kanal gehaltene Spitze des Katheters gelenkt wird. Insbesondere bei dünnen und durchsichtigen Kathetern macht sich diese Einfädelhilfe für den Anwender als Erleichterung bemerkbar, die ihn entlastet und den Katheterisierungsvorgang beschleunigt. Die Beschleunigung des Vorgangs zwischen der Punktion und dem Anschluß eines Überleitungssystems an das distale Katheterende wirkt sich günstig auf den Patienten aus, weil die Traumatisierung des Gewebes gering gehalten, Infektionsgefahr an der Punktionsstelle bis zu ihrem sterilen Verschluß vermindert und die medikamentöse Versorgung des Patienten durch den Katheter rasch gewährleistet wird.

Das längsteilbare Kopfstück kann aus zwei lösbar zusammengeschlossenen Teilen eines beliebig geformten Kör pers bestehen, die klappbar derart miteinander verbunden sind, daß der Kanal auf seiner ganzen Länge geöffnet werden kann, damit das Kopfstück von der endseitig koaxial zusammengefügten Führungsdraht-Kathetereinheit radial abgenommen, und der Katheter ohne Kopfstück über den Führungsdraht feinfühlig zum Zielort geführt werden kann.

In vorteilhafter Ausgestaltung der Erfindung ist vorgesehen, daß das Kopfstück aus zwei identischen Halbschalen besteht, die zu einem rohrformigen Körper zusammengefügt und von einem abziehbaren koaxialen Ring zusammengehalten sind. Die beiden Halbschalen sind vorzugsweise jeweils als separate Teile aus Kunststoff gefertigt und können zusammengreifende Vorsprünge bzw. Vertiefungen aufweisen, die die beiden Halbschalen so zueinander ausrichten, daß der gerade Kanal verkantungsfrei und vorsprungslos ist, wenn der Ring aufgesteckt ist und das Kopfstück eine symmetrische Einheit bildet. Jede Halbschale kann eine nach außen gerichtete Grifflasche aufweisen und bei zusammengestecktem Kopfstück sind vorteilhafterweise die beiden Grifflaschen in gleicher Ebene nach entgegengesetzten Seiten gerichtet, so daß nach Abziehen des Ringes mit jeder Hand eine Grifflasche erfaßt und durch Bewegung der Halbschalen in entgegengesetzte Richtungen das Kopfstück auf einfache Weise längsgeteilt werden kann.

Zur Verkürzung der axialen Länge des Kopfstückes befinden sich zweckmäßigerweise die beiden Grifflaschen auf der einen Querhälfte des Kopfstückes, während die andere Querhälfte als zylindrischer Stutzen zur Aufnahme des Ringes ausgebildet ist. Dabei ist es notwendig, daß die Grifflaschen in der Querhälfte des Kopfstückes an geordnet sind, die den trichterförmigen Abschnitt des Kanals aufweist, damit zur Auseinandernahme des Kopfstückes bei eingefädeltem Führungsdraht der Ring nach hinten, d.h. gegen den Anwender, frei abgezogen werden kann. Diese Anordnung ermöglicht im übrigen die Befestigung eines Schutzschlauches für den Katheter an dem Ring. Der aus einer dünnen Folie bestehende Schutzschlauch umgibt den mit seiner Spitze in den zylindrischen Abschnitt des Kanals des Kopfstückes lösbar eingeklemmten Katheter über seine ganze Länge, und er ist auch während der Applikation des Katheters vorhanden, nachdem das Kopfstück abgenommen und verworfen worden ist. Der Katheter wird durch den Schutzschlauch hindurch erfaßt und auf dem eingefädelten Führungsdraht vorgeschoben, wobei der Schutzschlauch ziehharmonikaartig zusammengeschoben wird und sich nach Loslassen wieder streckt. Nach Beendigung des Vorschiebens des Katheters zum Zielort wird der Führungsdraht in den Schutzschlauch hineingezogen und mit diesem entsorgt. Der Schutzschlauch ist zweckmäßigerweise am hinteren Ende offen, damit der Führungsdraht nach Aufschieben des Katheters dort austreten kann.

Die Innenfläche des zylindrischen Abschnittes des Kanals des Kopfstückes ist mit einer umfangsmäßigen Rillenprofilierung versehen, die dazu dient, die Einklemmung der Katheterspitze zwischen den beiden Halbschalen des Kopfstückes zu verbessern, ohne ihn zusammenzuquetschen, so daß dem durch den trichterförmigen Abschnitt des Kanals einzuführenden Führungsdraht das freie Katheterlumen offen gegenüberliegt. Um ein Anstoßen des Führungsdrahtendes gegen die Stirnfläche der Wand des Katheters zu vermeiden, ist zweckmäßigerweise der Durchmesser des zylindrischen Abschnittes des Kanals um die Wandstärke des Katheters größer als die kleine Öffnung des trichterförmigen Abschnittes, die in den zylindrischen Abschnitt mündet. Die Katheter-Stirnfläche liegt gegen die auf diese Weise gebildete Schulter im Kanal an und es ergibt sich ein glattwandiger Durchlaß für den Führungsdraht.

In der Zeichnung ist ein Ausführungsbeispiel der Vorrichtung schematisch dargestellt.

Es zeigen:

Fig. 1 eine perspektivische Ansicht der kompletten Vorrichtung bei Beginn des Einfädelvorganges und

Fig. 2 eine perspektivische Ansicht der beiden Halbschalen des Kopfstückes vor Einlegen des Katheters.

Die Vorrichtung zum Einfädeln eines Führungsdrahtes 10, der als Seldinger-Spirale oder in anderer Weise als langgestreckte flexibel biegbare Führungssonde ausgebildet ist, besteht im wesentlichen aus einem Kopfstück 11, das aus zwei Halbschalen 12, 13 aus Kunststoff zusammengesetzt ist und einen axial durchgehenden geraden Kanal 14 enthält, der auf der Längsmittelachse des Kopfstückes 11 liegt. Die beiden Halbschalen 12 und 13 sind identisch. Ihre Ausbildung wird deshalb im wesentlichen unter Bezug auf die eine Halbschale 12 veranschaulicht.

Jede Halbschale 12, 13 hat halbkreisförmigen Querschnitt und sie werden mit ihren ebenen Flächen 15 auf einandergelegt, so daß sie einen zylindrischen rohrförmigen Körper bilden, dessen erste Querhälfte als zylindrischer Stutzen 16 geformt ist, und dessen zweite Querhälfte mit zwei in Achsebene nach entgegengesetzten Richtungen weisenden flachen Grifflaschen 17, 18 besetzt ist. Die außen abgerundete Grifflasche 17 ist an die Halbschale 12 so angeformt, daß ihre eine Fläche mit der ebenen Fläche 15 der Halbschale 12 bündig verläuft und die Grifflasche 18 ist entsprechend an der Halbschale 13 angebracht. An der Grenze zwischen den beiden Querhälften jeder Halbschale 12, 13 befindet sich ein halbkreisförmiger Kragen, die sich bei zusammengesetztem Kopfstück 11 zu einem kreisförmigen Ringbund 20 ergänzen.

Auf der Außenfläche der ersten Querhälfte sind zu ihrer ebenen Stirnfläche 23 parallele, breite Rippen 21 und Rillen 22 ausgebildet, die sich bei zusammengesetztem Kopfstück 11 zu geschlossenen Partien ergänzen.

In der ebenen Fläche 15 jeder Halbschale 12 bzw. 13 des Kopfstückes 11 sind zur Bildung des an beiden Enden offenen Kanals 14 koaxial jeweils eine umfangsmäßige Hälfte eines trichterförmigen Abschnittes 30, eines geraden zylindrischen Abschnittes 31 und eines konischen Abschnittes 32 ausgebildet. Der trichterförmige Abschnitt 30 erstreckt sich von dem proximalen äußeren Rand der Halbschale 12 bzw. 13 etwa bis zum Ende der ersten Querhälfte. Hier mündet seine kleine Öffnung 35 in den koaxialen zylindrischen Abschnitt 31, dessen Durchmesser etwas größer ist als die Öffnung 35, so daß eine Schulter 36 entsteht. Die radiale Breite der Schulter 36 entspricht im wesentlichen der Wandstärke eines Katheters 40, dessen Spitze 41 in den zylindri schen Abschnitt 31 so eingefügt wird, daß ihre Stirnfläche gegen die Schulter 36 anliegt. Der Durchmesser des zylindrischen Abschnittes 31 ist dem Außendurchmesser des Katheters 40 angepaßt. Die Innenfläche des zylindrischen Abschnittes 31 des Kanals 14 ist mit einer Gewindeprofilierung 37 versehen, die die klemmende Festhaltung der Katheterspitze 41 zwischen den beiden Halbschalen 12, 13 verbessern soll.

Zur genauen Zusammenpassung der beiden Halbschalen 12, 13 dienen zwei Stifte 38 und zwei Vertiefungen 39, die paarweise neben dem Kanal 14 angeordnet sind und jeweils mit dem Gegenelement an der aufgesetzten Halbschale 12 bzw. 13 zusammengreifen. Zum Zusammenhalt der beiden Halbschalen 12, 13 ist ein kreisförmiger Ring 45 vorgesehen, der auf den zylindrischen Stutzen 16 des Kopfstückes 11 bis zum Anschlag gegen den Ringbund 20 aufgeschoben wird. Die axiale Länge des Ringes 45 ist vorzugsweise etwas größer als diejenige des Stutzens 16 des Kopfstückes 11 und zur Erleichterung der Handhabung weist er radial gerichtete Flügel 46 auf, deren Ebene in seiner Längsachse verläuft. An das dem Kopfstück 11 abgewandte Ende des Ringes 45 ist ein Schutzschlauch 47 aus dünner Folie angesetzt, dessen Länge etwa der Länge des Katheters 40 entspricht bzw. geringfügig über dessen Ende übersteht. Das hintere Ende 48 des Schutzschlauches 47 ist offen oder er ist so lang, daß er den Seldingerdraht 10 in ganzer Länge aufnehmen kann.

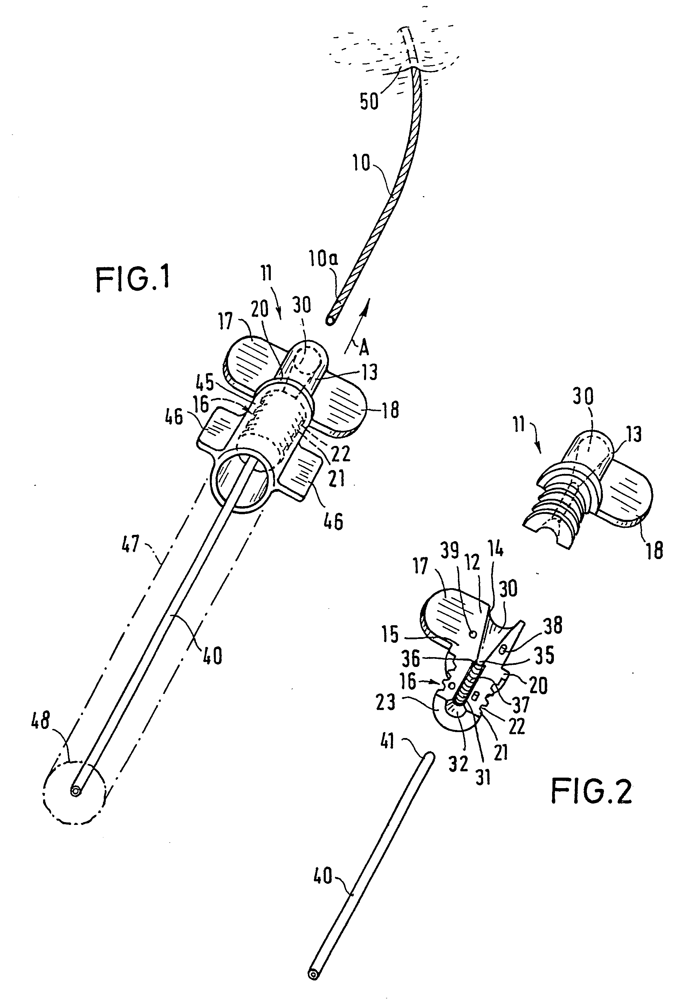

Die Vorrichtung wird mit in den zylindrischen Abschnitt 31 des Kanals 14 des Kopfstückes 11 eingeklemmtem Katheter 40 und aufgesteckter Ring-Schutzschlauchanordnung 45, 47 von dem Anwender an den Flügeln 46 erfaßt und in Richtung des Pfeiles A (Fig. 1) gegen das extra korporale Ende 10a des Führungsdrahtes 10 geführt, dessen Hauptlänge in Gewebe 50 steckt und zu einem Zielort für den Katheter 40 verläuft. Das freie Ende 10a des Führungsdrahtes 10 gleitet auf der glatten Fläche des trichterförmigen Abschnittes 30 entlang und wird unmittelbar in die axiale Öffnung der Spitze 41 des Katheters 40 hineingelenkt. Das bisher umständliche Einfädeln von Führungsdraht und Katheter kann mit Hilfe des trichterförmigen Abschnittes 30 des Kopfstückes 11 blind erfolgen. Nachdem der Katheter 40 auf das Ende 10a des Führungsdrahtes 10 aufgefädelt ist, wird der Ring 45 von dem Stutzen 16 axial abgezogen, so daß das Kopfstück 11 in seine beiden Halbschalen 12 und 13 zerlegbar ist und der Katheter 40 von dem Kopfstück 11 freikommt. Unter Belassung des Schutzschlauches 47 auf dem Katheter 40 wird dieser über den Führungsdraht 10 in das Gewebe 50 eingeschoben und bis zum Zielort bewegt.

Nach Beendigung der Kathetereinführung tritt das Ende 10a des Führungsdrahtes 10 aus dem offenen Ende 48 des Schutzschlauches 47 aus, und beide Teile können in ihrer Eigenschaft als Einmalprodukt gemeinsam weggeworfen werden. Das distale Ende des verlegten Katheters 40 wird mit einem Anschlußstück zur Ankupplung eines Überleitungssystems ausgerüstet.

## Ansprüche

1. Vorrichtung zum Einfädeln eines Führungsdrahtes (10) in einen Katheter (40), **gekennzeichnet durch** ein längsteilbares Kopfstück (11) mit einem axial durchgehenden geraden Kanal (14), der an einem Ende einen zylindrischen Abschnitt (31) aufweist, in welchem die Spitze des Katheters (40) lösbar eingeklemmt ist und der am anderen Ende mit einem trichterförmigen Abschnitt (30) versehen ist, dessen größere Öffnung nach außen gerichtet ist.

2. Vorrichtung nach Anspruch 1, **dadurch gekennzeichnet,** daß der Durchmesser des zylindrischen Abschnittes (31) des Kanals (14) des Kopfstückes (11) um die Wandstärke des Katheters (40) größer ist als die kleine Öffnung (35) des trichterförmigen Abschnittes (30) des Kanals (14).

3. Vorrichtung nach Anspruch 1 oder 2, **dadurch gekennzeichnet,** daß die Innenfläche des zylindrischen Abschnittes (31) des Kanals (14) des Kopfstückes (11) mit einer umfangsmäßigen Rillenprofilierung (37) versehen ist.

4. Vorrichtung nach einem der Ansprüche 1 bis 3, **dadurch gekennzeichnet,** daß das Kopfstück (11) aus zwei identischen Halbschalen (12, 13) besteht, die zu einem rohrformigen Körper zusammengefügt und von einem abziehbaren koaxialen Ring (45) zusammengehalten sind.

5. Vorrichtung nach Anspruch 4, **dadurch gekennzeichnet,** daß jede Halbschale (12, 13) eine nach außen gerichtete Grifflasche (17, 18) aufweist und daß bei zusammengestecktem Kopfstück (11) die beiden Grifflaschen (17, 18) in gleicher Ebene nach entgegengesetzten Seiten gerichtet sind.

6. Vorrichtung nach Anspruch 5, **dadurch gekennzeichnet,** daß sich die beiden Grifflaschen (17, 18) auf der einen Querhälfte des Kopfstückes (11) befinden und das die andere Querhälfte als zylindrischer Stutzen (16) zur Aufnahme des Ringes (45) ausgebildet ist.

7. Vorrichtung nach einem der Ansprüche 4 bis 6, **dadurch gekennzeichnet,** daß an dem Ring (45) ein an beiden Enden offener Schutzschlauch (47) befestigt ist.

FIG.1

FIG.2